# EUROPÄISCHE PATENTANMELDUNG

(11) **EP 1 516 921 A1**
(43) Veröffentlichungstag der Anmeldung: **23.03.2005**
(21) Anmeldenummer: 04019375.7
(22) Anmeldetag: 16.08.2004
(51) Int. Cl.: C12M 1/107, C12M 1/113, C12P 5/02, C02F 11/04, B01F 5/10, B01F 15/02

(54) **Fermentationsanlage**

(30) Priorität: 19.09.2003 AT 14882003
(71) Anmelder: GE Jenbacher GmbH & Co. OHG, 6200 Jenbach (AT)
(72) Erfinder: Schiliro, Michele, 6200 Jenbach (AT); Häusl, Günther, 6200 Jenbach (AT)
(74) Vertreter: Torggler, Paul N.

(57) **Zusammenfassung**

Fermentationsanlage zur gemeinsamen Fermentierung eines Basissubstrats (8) und mindestens eines Co-Substrates (1), wie zum Beispiel Mais, Raps, Silage oder dergleichen, mit einem Fermentationsbehälter (5) und einem im Wesentlichen geschlossenen Co-Substrat-Kanal (15) mit darin angeordneter Fördereinrichtung zum Einbringen des Co-Substrates (1) in den Fermentationsbehälter (5), wobei im bzw. am Co-Substrat-Kanal (15) mindestens eine Mischeinrichtung (4) zum Vermischen des Co-Substrates mit dem Basissubstrat angeordnet ist.

## Beschreibung

Die vorliegende Erfindung betrifft eine Fermentationsanlage zur gemeinsamen Fermentierung eines Basissubstrats und mindestens eines Co-Substrates, wie zum Beispiel Mais, Raps, Silage oder dergleichen, mit einem Fermentationsbehälter und einem im wesentlichen geschlossenen Co-Substrat-Kanal mit darin angeordneter Fördereinrichtung zum Einbringen des Co-Substrates in den Fermentationsbehälter.

Die Verwendung von Fermentationsanlagen zur Gewinnung von methanhaltigen Gasen ist beim Stand der Technik bekannt. Meist wird in einem Fermentationsbehälter das Basissubstrat (zum Beispiel Gülle oder dergleichen) mit Verweilzeiten von ca. 20 bis 50 Tagen auf einer Temperatur von 37° C unter gelegentlichem Umrühren gehalten, wobei ein Gasgemisch aus Methan, Kohlendioxid, Wasserstoff sowie Schwefelwasserstoff und anderen Spurengasen entsteht, welches anschließend in bekannter Weise gereinigt und zum Beispiel zur Verbrennung in Gasmotoren verwendet wird. Um den Gasertrag zu erhöhen, werden häufig sogenannte Co-Substrate in Form von Mais, Raps, Silage oder dergleichen dem Basissubstrat beigemengt.

Die EP 1 162 260 A2 zeigt eine entsprechende Fermentationsanlage, bei der das Co-Substrat mittels einer Förderschnecke über eine Einwurfluke des Fermentationsbehälters gefördert wird, um von dort frei auf die Oberfläche des Basissubstrats zu fallen. Die hier gezeigte Anlage hat den wesentlichen Nachteil, dass das Co-Substrat sich an der Einwurfstelle ansammelt und nur sehr schlecht und langsam unter das Basissubstrat untergemischt wird.

Eine gattungsgemäße Fermentationsanlage ist auch aus der EP 1 170 357 A1 bekannt. Hier dient als Fördereinrichtung eine Kolbenpresse. Diese schiebt das Co-Substrat durch einen Co-Substrat-Kanal in den Fermentationsbehälter. Optional ist in dem Co-Substrat-Kanal eine Wasser-Spüldüse zur Verbesserung der Förderfähigkeit vorgesehen. Die JP 2002-068483 zeigt einen Trichter mit daran anschließendem Co-Substrat-Kanal, wobei als Fördereinrichtung eine Schnecke teils im Co-Substrat-Kanal und teils im Trichter angeordnet ist.

Sowohl bei der Anlage gemäß der EP 1 170 357 A1 als auch bei Verwendung der aus der JP 2002-068483 bekannten Einrichtung tritt das oben geschilderte Problem, dass sich das Co-Substrat an der Einwurfstelle ansammelt und nur schlecht untergemischt wird, ebenfalls auf.

Aufgabe der vorliegenden Erfindung ist es, den genannten Nachteil zu beseitigen.

Dies wird erfindungsgemäß dadurch erreicht, dass im bzw. am Co-Substrat-Kanal mindestens eine Mischeinrichtung zum Vermischen des Co-Substrates mit dem Basissubstrat angeordnet ist.

Durch die erfindungsgemäße Mischeinrichtung im oder am Co-Substrat-Kanal ist es möglich, das Co-Substrat in einer bereits gut mit dem Basissubstrat vermischten Form in den Fermentationsbehälter einzubringen, sodass der anaerobe Fermentationsprozess möglichst rasch und vollständig einsetzen kann.

Als Co-Substrat können neben den bereits genannten Mais, Raps und anderen Silagen bevorzugt auch andere nachwachsende Rohstoffe eingesetzt werden.

Ein günstiges Verfahren zum Einbringen mindestens eines Co-Substrates in einen mit Basissubstrat füllbaren Fermentationsbehälter sieht vor, dass das Co-Substrat von einer Fördereinrichtung zu einer Mischeinrichtung gefördert wird und vor dem Einbringen in das in dem Fermentationsbehälter angeordnete Basissubstrat von der Mischeinrichtung einer Teilmenge des Basissubstrates zugemischt wird. Diese Teilmenge des Basissubstrates wird günstigerweise vor dem Zumischen zum Co-Substrat dem Fermentationsbehälter entnommen.

Die beim Stand der Technik bekannten Fermentationsanlagen haben in der Regel auch den Nachteil, dass Gase beim Eindringen des Co-Substrates in den Fermentationsbehälter aus diesem entweichen können, was sowohl zur Geruchsbelästigung als auch zur Freisetzung von brennbaren Gasen in die Umgebung führt. Um dies zu verhindern, sieht eine günstige Ausführungsform der Erfindung vor, dass der Fermentationsbehälter und der Co-Substrat-Kanal eine im Wesentlichen gasdichte Einheit bilden.

In einer bevorzugten Variante kann die Fermentationsanlage auch teil- oder vollautomatisch betrieben werden, wobei hierzu eine Steuerung oder eine Regelung zum automatischen Betrieb der Fördereinrichtung und/oder der Mischeinrichtung vorgesehen ist. Im Falle einer Regelung können verschiedene, gemäß dem Stand der Technik messbare, physikalische oder chemische Parameter als Regelgrößen herangezogen werden.

Ein weiterer Aspekt der Erfindung sieht einen im Wesentlichen geschlossenen Co-Substrat-Kanal zum Einbringen von Co-Substraten in einen mit Basissubstrat füllbaren Fermentationsbehälter vor, wobei eine Fördereinrichtung für Co-Substrat und eine Mischeinrichtung zum Vermischen von Co-Substrat mit dem im Fermentationsbehälter vorhandenen Basissubstrat, vorzugsweise hintereinander, im bzw. am Co-Substrat-Kanal angeordnet sind.

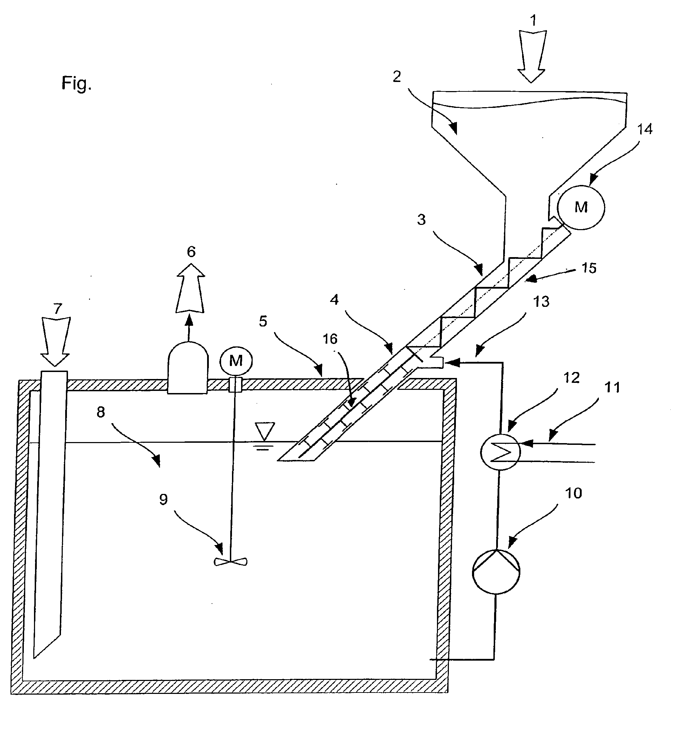

Weitere Einzelheiten und Merkmale der vorliegenden Erfindung ergeben sich aus der Beschreibung der beiliegenden Figur.

Diese zeigt schematisch eine erfindungsgemäße Fermentationsanlage mit einem Fermentationsbehälter 5 und einem Co-Substrat-Kanal 15. In den Fermentationsbehälter 5 wird über den Basissubstrateintrag 7 Basissubstrat 8 eingebracht. Über einen nicht dargestellten, aber beim Stand der Technik bekannten, Abzug wird verbrauchtes Substrat aus dem Fermentationsbehälter in einer Menge wieder abgezogen, bei der Eintrag und Austrag sich ungefähr die Waage halten und die schematisch dargestellte Füllhöhe zumindest ungefähr konstant bleibt. Das motorbetriebene Rührwerk 9 ist für die Durchmischung der einzelnen Substanzen innerhalb des Fermentationsbehälters vorgesehen. Der Abzug des entstandenen Biogasgemisches erfolgt über die Biogasentnahme 6.

Das Co-Substrat wird über den Co-Substrat-Kanal 15 in das Basissubstrat im Fermentationsbehälter 5 eingebracht. Der Co-Substrat-Kanal 15 ist hierbei günstigerweise als ein im Wesentlichen geschlossenes Rohr ausgebildet, in dem eine Fördereinrichtung 3, zum Beispiel in Form einer Förderschnecke, Co-Substrat 1 aus dem Vorlagetrichter 2 zur erfindungsgemäßen Mischeinrichtung 4 transportiert. Die Mischeinrichtung 4 weist zum einen einen Rührer 16 auf. Zum anderen ist in diesem Ausführungsbeispiel aber auch eine im Betrieb zumindest zeitweise bzw. intermittierend basissubstratführende Verbindungsleitung zwischen dem Fermentationsbehälter 5 und dem Co-Substrat-Kanal 15 vorgesehen. Zur Förderung des Basissubstrats 8 ist in dieser Verbindungsleitung 13 eine Pumpe 10 angeordnet. Die optimale Betriebstemperatur von ca. 37° C wird über eine des weiteren in der Verbindungsleitung angeordnete Heizeinrichtung 12, hier in Form eines Wärmetauschers mit entsprechender Wärmeanbindung 11, gewährleistet. Die Verbindungsleitung 13 mündet im Anschluss an das zum Fermentationsbehälter 5 weisende Ende der Fördereinrichtung 3 in den Co-Substrat-Kanal 15. In der so ausgebildeten Mischeinrichtung wird das Co-Substrat 1 mit dem über die Verbindungsleitung 13 eingespeisten Basissubstrat innig vermischt bevor es in das im Fermentationsbehälter 5 anstehende Basissubstrat 8 eingebracht wird. Die dargestellte Mischeinrichtung 4, bei der sowohl Basissubstrat zugegeben wird, als auch ein zusätzliches Verrühren von Co-Substrat 1 und Basissubstrat 8 stattfindet, ist eine besonders bevorzugte Lösung. Einfachere Ausführungsformen können auch vorsehen, dass im Co-Substrat-Kanal 15 oder an dessen in das Basissubstrat reichenden Ende lediglich ein Rührer 16 vorgesehen und auf die Verbindungsleitung 13 vollständig verzichtet ist. Alternativ kann der Rührer 16 bei einer entsprechenden Förderleistung der Pumpe 10 auch entfallen, solange genug Basissubstrat durch die Verbindungsleitung 13 und den Co-Substrat-Kanal 15 gepumpt wird. In diesem Fall besteht die Mischeinrichtung aus dem Teil des Co-Substrat-Kanals 15, in dem das Basissubstrat 8 über die Verbindungsleitung 13 in das Co-Substrat 1 eingespült wird, sowie aus Verbindungsleitung 13 und Pumpe 10 und gegebenenfalls Heizeinrichtung 12.

Grundsätzlich können die Fördereinrichtung 3 und Mischeinrichtung 4 eigene voneinander getrennte Antriebe aufweisen. Im gezeigten Ausführungsbeispiel sind die Förderschnecke der Fördereinrichtung 3 und der Rührer 16 der Mischeinrichtung 4 hintereinander auf einer gemeinsamen Antriebswelle angeordnet, sodass sie von einem gemeinsamen Antriebsmotor 14 gedreht werden können.

Der Füllstand im Fermentationsbehälter 5 wird günstigerweise in der Weise eingeregelt, dass das untere Ende des Co-Substrat-Kanals 15 in das Basissubstrat 8 eintaucht. Alternativ kann zum Eintauchen des unteren Endes des Co-Substrat-Kanals 15 dieser auch höhenverstellbar ausgeführt sein. Ist das Eintauchen nicht gegeben, so muss eine entsprechende Förderleistung der Pumpe 10 vorgesehen sein, sodass ausreichend Basissubstrat zum Untermischen im Co-Substrat-Kanal vorhanden ist. Die optimale Betriebstemperatur von 37° C wird im gezeigten Ausführungsbeispiel durch die Heizeinrichtung 12 gewährleistet. Alternativ oder zusätzlich kann auch noch eine zusätzliche, beim Stand der Technik bekannte weitere Heizeinrichtung (nicht dargestellt) im Fermentationsbehälter vorgesehen sein.

Die gezeigte Fermentationsanlage hat unter anderem den Vorteil, dass sie vollständig gasdicht ausgebildet werden kann. Hierdurch werden zum einen unnötige Verluste an Biogas sowie eine ungewünschte Geruchsbelästigung durch die Anlage vermieden. Darüber hinaus ist eine Gefährdung der Anlage bzw. deren Umgebung durch Austreten brennbarer Gase verhindert. Die gezeigte Anlage kann durch hier nicht dargestellte Steuer- oder Regeleinrichtungen vollständig oder teilweise automatisiert betrieben werden.

## Patentansprüche

1. Fermentationsanlage zur gemeinsamen Fermentierung eines Basissubstrats und mindestens eines Co-Substrates, wie zum Beispiel Mais, Raps, Silage oder dergleichen, mit einem Fermentationsbehälter und einem im Wesentlichen geschlossenen Co-Substrat-Kanal mit darin angeordneter Fördereinrichtung zum Einbringen des Co-Substrates in den Fermentationsbehälter, **dadurch gekennzeichnet, dass** im bzw. am Co-Substrat-Kanal (15) mindestens eine Mischeinrichtung (4) zum Vermischen des Co-Substrates mit dem Basissubstrat angeordnet ist.

2. Fermentationsanlage nach Anspruch 1, **dadurch gekennzeichnet, dass** die Mischeinrichtung im Anschluss an das zum Fermentationsbehälter (5) weisende Ende der Fördereinrichtung (3) und/oder direkt am in den Fermentationsbehälter (5) weisenden Ausgang des Co-Substrat-Kanals (15) angeordnet ist.

3. Fermentationsanlage nach Anspruch 1 oder 2, **dadurch gekennzeichnet, dass** die Fördereinrichtung (3) eine Förderschnecke aufweist.

4. Fermentationsanlage nach einem der Ansprüche 1 bis 3, **dadurch gekennzeichnet, dass** die Mischeinrichtung (4) einen Rührer (16) aufweist.

5. Fermentationsanlage nach einem der Ansprüche 1 bis 4, **dadurch gekennzeichnet, dass** die Fördereinrichtung (3) und die Mischeinrichtung (4) einen gemeinsamen Antrieb (14), vorzugsweise eine gemeinsame Antriebswelle, aufweisen.

6. Fermentationsanlage nach einem der Ansprüche 1 bis 5, **dadurch gekennzeichnet, dass** eine im Betrieb zumindest zeitweise basissubstratführende Verbindungsleitung (13) zwischen dem Fermentationsbehälter (5) und dem Co-Substrat-Kanal (15) vorgesehen ist, welche vorzugsweise im Anschluss an das zum Fermentationsbehälter (5) weisende Ende der Fördereinrichtung (3) in den Co-Substrat-Kanal (15) mündet.

7. Fermentationsanlage nach Anspruch 6, **dadurch gekennzeichnet, dass** in der Verbindungsleitung (13) eine Pumpe (10) angeordnet ist.

8. Fermentationsanlage nach Anspruch 6 oder 7, **dadurch gekennzeichnet, dass** in der Verbindungsleitung (13) eine Heizeinrichtung (12), vorzugsweise ein Wärmetauscher, angeordnet ist.

9. Fermentationsanlage nach einem der Ansprüche 1 bis 8, **dadurch gekennzeichnet, dass** der Fermentationsbehälter (5) und der Co-Substrat-Kanal (15) eine im Wesentlichen gasdichte Einheit bilden.

10. Fermentationsanlage nach einem der Ansprüche 1 bis 9, **dadurch gekennzeichnet, dass** eine Steuerung oder eine Regelung zum automatischen Betrieb der Fördereinrichtung (3) und/oder der Mischeinrichtung (4) vorgesehen ist.

11. Fermentationsanlage nach einem der Ansprüche 1 bis 10, **dadurch gekennzeichnet, dass** sie der Herstellung von Biogas dient.

12. Fermentationsanlage nach einem der Ansprüche 1 bis 11, **dadurch gekennzeichnet, dass** der Co-Substrat-Kanal zumindest bereichsweise als geschlossenes Rohr ausgebildet ist.

13. Im wesentlichen geschlossener Co-Substrat-Kanal zum Einbringen von Co-Substraten in einen mit Basissubstrat füllbaren Fermentationsbehälter, **dadurch gekennzeichnet, dass** eine Fördereinrichtung (3) für Co-Substrat und eine Mischeinrichtung (4) zum Vermischen von Co-Substrat mit dem im Fermentationsbehälter (5) vorhandenen Basissubstrat, vorzugsweise hintereinander, im bzw. am Co-Substrat-Kanal (15) angeordnet sind.

14. Verfahren zum Einbringen mindestens eines Co-Substrates in einen mit Basissubstrat füllbaren Fermentationsbehälter, **dadurch gekennzeichnet, dass** das Co-Substrat (1) von einer Fördereinrichtung (3) zu einer Mischeinrichtung (4) gefördert wird und vor dem Einbringen in das in dem Fermentationsbehälter (5) angeordnete Basissubstrat (8) von der Mischeinrichtung (4) einer Teilmenge des Basissubstrates (8) zugemischt wird.

15. Verfahren nach Anspruch 14, **dadurch gekennzeichnet, dass** die Teilmenge des Basissubstrates (8) vor dem Zumischen des Co-Substrates (1) dem Fermentationsbehälter entnommen wird.
